# EUROPEAN PATENT SPECIFICATION

(11) **EP 1 097 153 B1**
(45) Date of publication and mention of the grant of the patent: **01.10.2003**
(21) Application number: 99941925.2
(22) Date of filing: 15.07.1999
(51) Int. Cl.: C07D 487/04, A61K 31/505, A61K 31/41

(54) **NOVEL TRIAZOLO[4,5-D]PYRIMIDINE COMPOUNDS**
TRIAZOLO[4,5-D]PYRIMIDIN-VERBINDUNGEN
COMPOSES TRIAZOLO[4,5-D]PYRIMIDINIQUES

(30) Priority: 17.07.1998 SE 9802574
(43) Date of publication of application: 09.05.2001
(73) Proprietor: AstraZeneca AB, 151 85 Södertälje (SE)
(72) Inventor: GUILE, Simon, Loughborough, Leics. LE11 5RH (GB); SPRINGTHORPE, Brian, Loughborough, Leics. LE11 5RH (GB)
(86) International application number: SE9901277
(87) International publication number: WO00004021

(56) References cited:
- WO-A1-97/03084
- WO-A1-98/28300
- WO-A1-99/05142

## Description

The present invention provides new triazolo[4,5-*d*]pyrimidine compounds, their use as medicaments, compositions containing them and processes for their preparation.

Platelet adhesion and aggregation are initiating events in arterial thrombosis. Although the process of platelet adhesion to the sub-endothelial surface may have an important role to play in the repair of damaged vessel walls, the platelet aggregation that this initiates can precipitate acute thrombotic occlusion of vital vascular beds, leading to events with high morbidity such as myocardial infarction and unstable angina. The success of interventions used to prevent or alleviate these conditions, such as thrombolysis and angioplasty is also compromised by platelet mediated occlusion or re-occlusion.

A number of converging pathways lead to platelet aggregation. Whatever the initial stimulus, the final common event is a cross linking of platelets by binding of fibrinogen to a membrane binding site, glycoprotein IIb/IIIa (GPIIb/IIIa). The high anti-platelet efficacy of antibodies or antagonists for GPIIb/IIIa is explained by their interference with this final common event. However, this efficacy may also explain the bleeding problems that have been observed with this class of agent. Thrombin can produce platelet aggregation largely independently of other pathways but substantial quantities of thrombin are unlikely to be present without prior activation of platelets by other mechanisms. Thrombin inhibitors such as hirudin are highly effective anti-thrombotic agents, but again may produce excessive bleeding because they function as both anti-platelet and anti-coagulant agents (The TIMI 9a Investigators (1994), *Circulation* **90**, pp. 1624-1630; The Global Use of Strategies to Open Occluded Coronary Arteries (GUSTO) IIa Investigators (1994) *Circulation* **90**, pp. 1631-1637; Neuhaus K.L. et. al. (1994) *Circulation* **90**, pp.1638-1642).

It has been found that ADP acts as a key mediator of thrombosis. A pivotal role for ADP is supported by the fact that other agents, such as adrenaline and 5-hydroxytryptamine (5HT, serotonin) will only produce aggregation in the presence of ADP. The limited anti-thrombotic efficacy of aspirin may reflect the fact that it blocks only one source of ADP which is that released in a thromboxane-dependent manner following platelet adhesion (see e.g. Antiplatelet Trialists' Collaboration (1994), *Br. Med. J*. **308**, pp. 81-106; Antiplatelet' Trialists'Collaboration (1994), *Br. Med. J.* **308**, pp.159-168). Aspirin has no effect on aggregation produced by other sources of ADP, such as damaged cells or ADP released under conditions of turbulent blood flow. ADP-induced platelet aggregation is mediated by the P_{*2T*}-receptor subtype uniquely located on the platelet membrane. Recently it has been shown that antagonists at this receptor offer significant improvements over other anti-thrombotic agents Accordingly there is a need to find P_{*2T*}-antagonists as anti-thrombotic agents.

WO 9828399A discloses triazolo[4,5-d]pyrimidine compounds substituted in the 3 position with a 2,3-dihydroxcyclopentane carboxamide group, in the 5 position with an alkyl thiol group and in the 7 position with a monosubstituted amino group. The compounds are useful in the treatment of platelet aggregation disorders.

WO9703084A discloses triazolo[4,5-d]pyrimidine compounds substituted in the 3 position with a 2,3-dihydroxcyclopentane group (substituted in the 1 position with a carboxylic acid derivative substituted ethyl or ethenyl group), in the 5 position with an alkyl thiol group and in the 7 position with a disubstituted amino group. The compounds are useful in the treatment of platelet aggregation disorders.

It has now been found that a series of triazolo[4,5-*d*]pyrimidine derivatives are P_{*2T*}-receptor antagonists. In a first aspect the invention therefore provides a compound of formula (I): 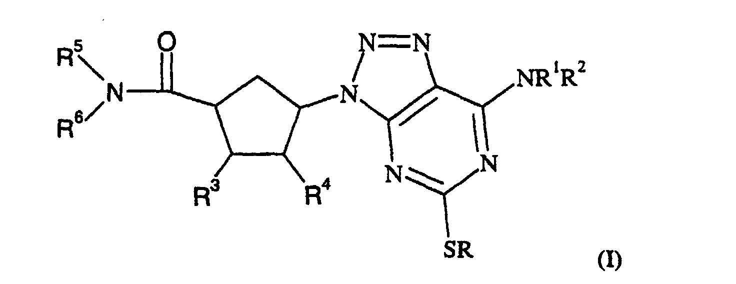 wherein:
R is C₁₋₆ alkyl, optionally substituted by one or more halogen, atoms
R¹ is C₁₋₄ alkyl;
R² is C₁₋₆-alkyl or a C₃₋₈-cycloalkyl group optionally substituted by phenyl optionally substituted by one or more substituents selected from halogen, SO₂NR⁸R⁹, and C₁₋₆ alkyl which is optionally substituted by one or more halogen atoms,
R³ and R⁴ are both hydroxy;
R⁵ is hydrogen or C₁₋₆ alkyl, and R⁶ is C₁₋₆ alkyl optionally substituted by
C₃₋₆-cycloalkyl or R⁶ is C₃₋₆-cycloalkyl or R⁵ and R⁶ together with the nitrogen atom to which they are attached form a 5- to 7-membered saturated ring;
R⁸, R⁹ are independently hydrogen or C₁₋₆ alkyl;
or a pharmaceutically acceptable salt or solvate thereof.

Alkyl groups, whether alone or as part of another group, can be straight chained or branched.

Preferably the compound of formula (I) has the following stereochemistry:

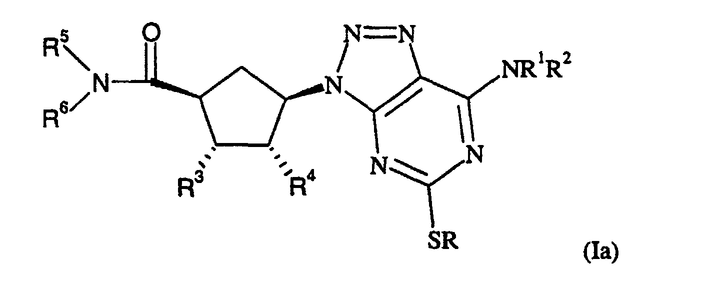

Preferably R is methyl or propyl.

Preferably R¹ is methyl.

Preferably R² is cyclopropyl optionally substituted by phenyl, 4-chlorophenyl, 3,4-difluorophenyl, 4-(aminosulphonyl)phenyl or 4-(methylaminosulphonyl)phenyl.

Preferably R⁵ is hydrogen.

Preferably R⁶ is C₁₋₆ alkyl optionally substituted by hydroxy or halogen. More preferably R⁶, ethyl, 2-hydroxyethyl or 2-fluoroethyl.

Particularly preferred compounds of the invention include:
[ 1*S*-[1α,2β,3β,4α(1*S**,2*R**)]]-*N*-Ethyl-2,3-dihydroxy-4-[7-[methyl(2-phenylcyclopropyl)amino]-5-(propylthio)-3*H*-[1,2,3]triazolo[4,5-*d*]pyrimidin-3-yl]cyclopentanecarboxamide,
[1*S*-[1α,2β,3β,4α(1*S**,2*R**)]]-*N*-Ethyl-2,3-dihydroxy-4-[7-[methyl(2-phenylcyclopropyl)amino]-5-(methylthio)-3*H*-[1,2,3]triazolo[4,5-*d*]pyrimidin-3-yl]cyclopentanecarboxamide,
[1*S*-[1α,2β,3β,4α(1*S**,2*R**)]]-2,3-Dihydroxy-*N*-(2-hydroxyethyl)-4-[7-[methyl(2-phenylcyclopropyl)amino]-5-(propylthio)-3*H*-[ 1 ,2,3]triazolo[4,5-*d*]pyrimidin-3-yl]cyclopentanecarboxamide,
[1*S*-[1α,2β,3β,4α(1*S**,2*R**)]]-2,3-Dihydroxy-4-[7-[methyl(2-phenylcyclopropyl)amino]-5-(propylthio)-3*H*-[ 1,2,3)triazolo[4,5-*d*)pyrimidin-3-yl]cyclopentanecarboxamide,
[1*S*-[1α,2β,3β,4α(1*S**,2*R**)]]-4-[7-[[2-(3,4-difluorophenyl)cyclopropyl]methylamino]-5-(propylthio)-3*H*-[1,2,3)triazolo[4,5-*d*)pyrimidin-3-yl]-*N*-ethyl 2,3-dihydroxycyclopentanecarboxamide,
[1*S*-[1α,2β,3β,4α( 1*S**,2*R**)]]-4-[7-[[2-(4-Chloropheny)cyclopropyl]methylamino]-5-(propylthio)-3*H* [1,2,3]triazolo[4,5-*d*]pyrimidin-3-yl]-*N*-ethyl-2,3-dihydroxycyclopentanecarboxamide,
[1*S*-[1α,2β,3β,4α(1*S**,2*R**)]]-4-[7-[[2-(4-Aminosulfonylphenyl)cyclopropyl]methylamino]-5-(propylthio)-3*H* [1,2,3]triazolo[4,5-*d*]pyrimidin-3-yl]-*N*-ethyl-2,3-dihydroxycyclopentanecarboxamide,
[1*S*-[1α,2β,3β,4α(1*S**,2*R**)]]-*N*-Ethyl-2,3-dihydroxy-4-[7-[methyl[2-(4-methylaminosulfonylphenyl)cyclopropyl]amino]-5-(propylthio)-3*H*-[ 1,2,3]triazolo[4,5-*d*]pyrimidin-3-yl]cyclopentanecarboxamide,
[1*S*-[1α,2β,3β,4α(1*S**,2*R**)]]-*N*-(2-Fluoroethyl)-2,3-dihydroxy-4-[7-[methyl(2-phenylcyclopropyl)amino]-5-(propylthio)-3*H* [1,2,3]triazolo[4,5-*d*]pyrimidin-3-yl]cyclopentanecarboxamide
or pharmaceutically acceptable salts or solvates thereof.

According to the invention there is further provided a process for the preparation of a compound of formula (I) which comprises reaction of a compound of formula (II): 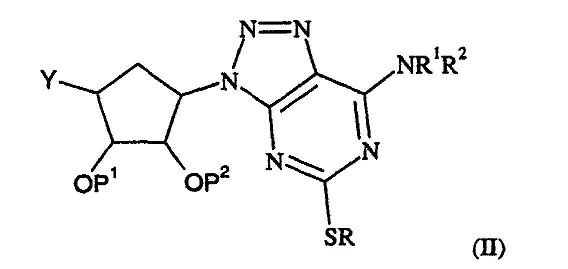 where R, R¹ and R² are as defined in formula (I) or are protected derivatives thereof, P¹ and P² are hydrogen or protecting groups and Y is CO₂H, with a compound of formula (III):

R⁵R⁶NH (III)

where R⁵ and R⁶ are as defined in formula (I), and optionally thereafter in any order
- converting one or more functional groups into a further functional group
- removing any protecting groups
- forming a pharmaceutically acceptable salt or solvate.

Examples of suitable groups which each P¹ and P² can independently represent are C₁₋₆-alkyl (preferably methyl), benzyl, (C₁₋₆-alkyl)₃Si (preferably trimethylsilyl) and a C(O)C₁₋₆-alkyl group (preferably acetyl). Preferably the two groups P¹ and P ² together with the atoms to which they are attached complete a ring, for example the two groups P¹ and P² together represent an alkylidene such as methylidene or, more preferably, isopropylidene, or an alkoxy methylidene such as ethoxymethylidene.

The reaction of compounds of formula (II) and (III) is preferably carried out in the presence of a coupling agent using methods known from peptide synthesis (see M. Bodanszky and A. Bodanszky, The Practice of Peptide Synthesis, Springer-Verlag, 1984). Suitable coupling agents include 1,1'-carbonyldiimidazole and dicyclohexylcarbodiimide; the preferred coupling agent is bromo-tris-pyrrolidino-phosphonium hexafluorophosphate or benzotriazole- 1 -yl-oxy-tris-(dimethylamino)phosphoniumhexafluorophosphate, used in the presence of *N,N*-diisopropylethylamine, The reaction is preferably carried out in *N,N*-dimethylformamide (DMF) or tetrahydrofuran (THF) and preferably at a temperature of from -15° to 120°C, more preferably at a temperature of from 0°C to room temperature.

Protecting groups can be added and removed using known reaction conditions. The use of protecting groups is fully described in Protective Groups in Organic Chemistry, edited by J W F McOmie, Plenum Press (1973), and Protective Groups in Organic Synthesis, 2nd edition, T W Greene & P G M Wutz, Wiley-Interscience (1991).

Alternative methods of activating a compound of formula (II) wherein Y is CO₂H include formation of an acyl halide or an acetic anhydride. Acid anhydrides may be formed by treatment with an acyl halide, such as acetyl chloride in the presence of a base, such as pyridine or by treatment with a dehydrating agent such as acetic acid anhydride or phosphorus pentoxide in an inert solvent. Acyl halides may be formed by treatment of the acid with a halogenating agent, for example P(III), P(V) or S(IV) halides such as phosphorus trichloride. Acyl halides may also be prepared by an exchange reaction of the acid with an acyl halide such as oxalyl bromide. The reactions may be performed in the halogenating agent or acyl halide as solvent or in other inert solvents such as methylene chloride, at a temperature of from 0 to 150°C. Activation is preferably carried out by treatment with oxalyl chloride in dichloromethane at room temperature.

Deprotection can be carried out using methods generally known in the art. For example for groups P¹/P² deprotection is preferably carried out as follows:
(i) where one or both of P¹ and P² represent C(O)C₁₋₆-alkyl, these groups can be removed by basic hydrolysis, for example by using a metal hydroxide, preferably an alkali metal hydroxide, such as sodium hydroxide or lithium hydroxide, or quaternary ammonium hydroxide in a solvent, such as aqueous ethanol or aqueous tetrahydrofuran, at a temperature of from 10° to 100°C, preferably the temperature is around room temperature; or by acidic hydrolysis using a mineral acid such as HCl or a strong organic acid such as trichloroacetic acid in a solvent such as aqueous 1,4-dioxane;
(ii) where one or both of P¹ and P² represent (C₁₋₆-alkyl)₃Si, these can be removed by the use of, for example, a fluoride ion source, for example tetra-n-butylammonium fluoride or hydrogen fluoride;
(iii) where one or both of P¹ and P² represent a C₁₋₆-alkyl group, these groups can be removed by the use of, for example, boron tribromide;
(iv) where one or both of P¹ and P² represent a benzyl group these can be removed by hydrogenolysis using a transition metal catalyst, for example palladium on charcoal, under an atmosphere of hydrogen, at a pressure of from 1 to 5 bar, in a solvent, such as acetic acid; and/or
(v) where both P¹ and P² together represent alkylidene or an alkoxy alkylidene, they can be removed by the use of, for example, a mineral or organic acid, preferably by using 2M aqueous hydrochloric acid in methanol/ 1,4-dioxane or aqueous trifluoroacetic acid at room temperature.

Compounds of formula (III) are commercially available.

A compound of formula (II) wherein Y is CO₂H, CONR⁵R⁶ or CO₂ R' can be prepared by reacting a compound of formula (IV): 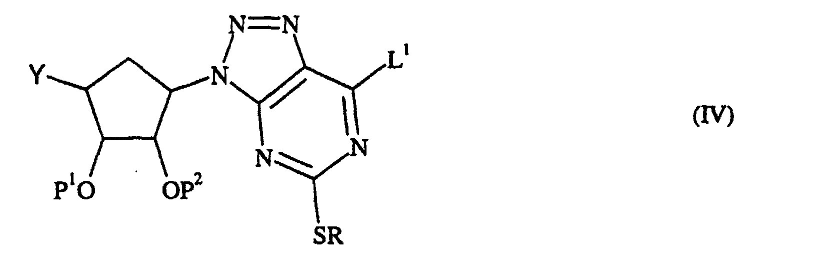 wherein R, P¹, P² R⁵ and R⁶ are as defined above, L¹ is a leaving group, for example a halogen atom and R' is a C₁₋₆-alkyl or benzyl group, with an amine NHR¹R² or a salt of NHR¹R² wherein R² is as defined above, in the presence of a base. Suitable salts of NHR¹R² include hydrochlorides. Suitable bases include an organic base such as triethylamine or an inorganic base such as potassium carbonate. The amines NHR¹R² can be prepared using procedures described in H Nishiyama *et al,* Bull. Chem. Soc., Jpn., 1995, **68**, 1247, P. Newman, Optical Resolution Procedures for Chemical Compounds, Vol. 1, Amines and Related Compounds; Optical Resolution and Information Centre: Manhattan College, Riverdale, NY, 1978, p120, J. Vallgarda *et al,* J. Chem. Soc. Perkin 1, 1994, 461. Certain amines NHR¹R² are novel compounds and form a further aspect of the invention.

A compound of formula (IV) can be prepared by diazotising a compound of formula (V): 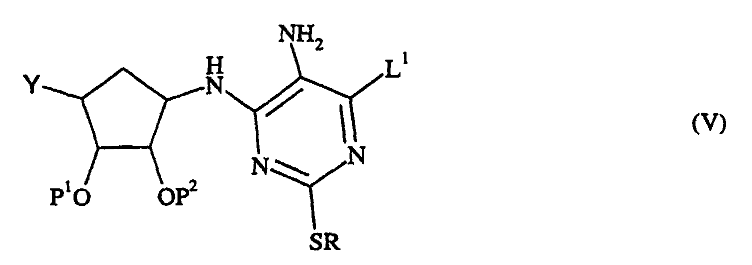 wherein R, Y, L¹, P¹ and P² are as defined above, with a metal nitrite, for example an alkali metal nitrite, especially sodium nitrite in dilute aqueous acid, for example 2M HCl, or with a C₁₋₆-alkyl nitrite in an inert solvent, at a temperature of from -20 to 100°C; preferred conditions are isoamyl nitrite in acetonitrile at 80°C.

A compound of formula (V) where Y is CO₂H can be prepared by reducing and hydrolysing a compound of formula (VI): 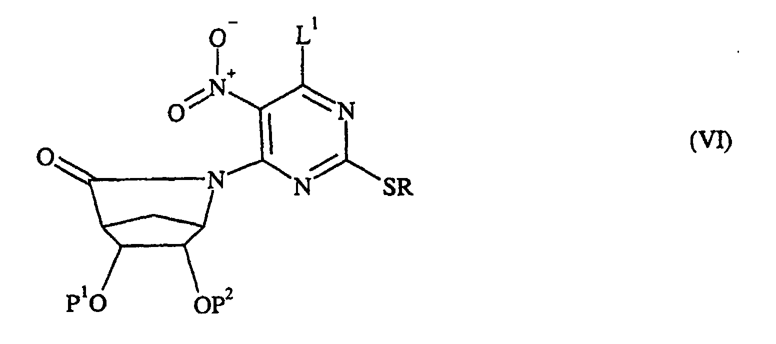 wherein R, L¹ , P¹ and P² are as defined above. The reduction may be carried out for example by using hydrogenation with a transition metal catalyst at a temperature around room temperature, for example palladium on charcoal under an atmosphere of hydrogen, preferably at a pressure from 1 to 5 atmospheres, in a solvent, for example ethanol, or by using iron in an acidic solvent such as acetic acid at a temperature of about 100°C.

To prepare a compound of formula (V) wherein Y is CO₂H, following the above reaction, hydrolysis of the compound derived from the compound of formula (VI) may be performed by using a mineral acid such as HCl or a strong organic acid such as trifluoroacetic acid in a solvent such as aqueous 1,4-dioxane, at a temperature of from 20 to 150°C. Preferably the reduction and hydrolysis are carried out simultaneously using iron in an acidic solvent, for example acetic acid, containing an alkaline earth metal halide, for example calcium chloride, at a temperature of about 80°C.

To prepare a compound of formula (V) wherein R⁹ is C₁₋₆-alkyl or benzyl, the compound of formula (VI) is treated with iron in acetic acid at a temperature of from 50 to 80°C so that the nitro group is reduced. The resulting intermediate is then treated with sodium borohydride in a mixture of water and C₁₋₆-alkyl alcohol or benzyl alcohol at around room temperature.

A compound of formula (VI) can be prepared by reacting a compound of formula (VII): 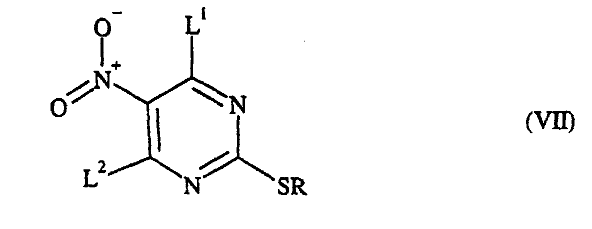 wherein L¹ and R are as defined above and L² is a leaving group, for example a halogen atom, wherein L¹ and L² are preferably the same, with a compound of formula (VIII): 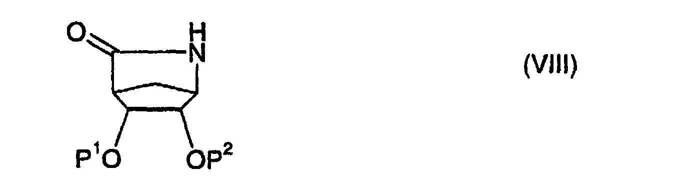 wherein P¹ and P² are as defined above, in the presence of a base such as C₁₋₆-alkyl-M or MH wherein M is a metal, for example butyl lithium, in an inert solvent, such as tetrahydrofuran (THF), at a temperature of from -10 to 100°C. Preferably sodium hydride is used in THF at room temperature. Preferably the compound of formula (VIII) has the following stereochemistry such that the above reactions give a compound of formula (la):

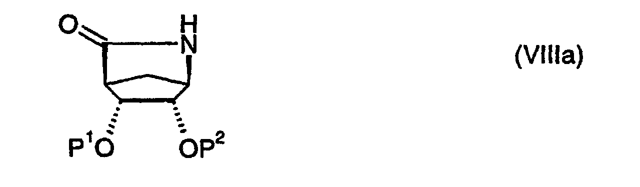

A compound of formula (VII) may be prepared from 4,6-dihydroxy-2-mercaptopyrimidine by alkylation with RL³ wherein R is as defined above and L³ is a suitable leaving group, for example a halogen atom, followed by nitration, whereafter the two alcohols are converted to leaving groups L¹ and L².

A compound of formula (V) where Y is CONR⁵R⁶ can be prepared by reacting a compound of formula (IX): 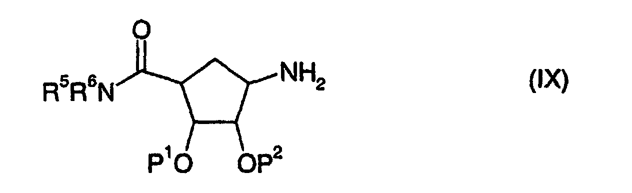 with a compound of formula (VII) where P¹, P², R¹, R⁵, R⁶, L¹, and L² are as defined above in a suitable solvent such as 1,4-dioxane in the presence of a base such as *N,N*-diisopropylethylamine followed by reduction of the nitro group. Compounds of formula (IX) may be prepared using procedures described in W09528160. Preferably the compound of formula (IX) has the following stereochemistry such that the above reactions give a compound of formula (Ia):

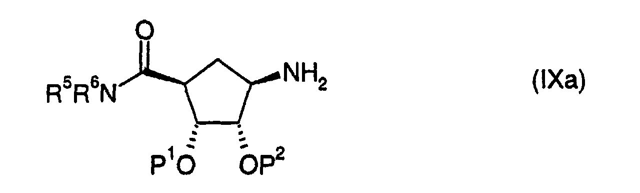

The group SR can be interconverted by oxidation of the sulphur, for example using oxone® or mCPBA, followed by treatment with a compound RSM where R is a different R group and M is a metal such as sodium.

All novel intermediates form a further aspect of the invention.

Salts of the compounds of formula (I) may be formed by reacting the free acid, or a salt thereof, or the free base, or a salt or a derivative thereof, with one or more equivalents of the appropriate base (for example ammonium hydroxide optionally substituted by C₁₋₆-alkyl or an alkali metal or alkaline earth metal hydroxide) or acid (for example a hydrohalic (especially HCI), sulphuric, oxalic or phosphoric acid). The reaction may be carried out in a solvent or medium in which the salt is insoluble or in a solvent in which the salt is soluble, e.g. water, ethanol, THF or diethyl ether, which may be removed *in vacuo,* or by freeze drying. The reaction may also be ametathetical process or it may be carried out on an ion exchange resin. The non-toxic physiologically acceptable salts are preferred, although other salts may be useful, e.g. in isolating or purifying the product.

The compounds of the invention act as P_{*2T*}-receptor antagonists. Accordingly, the compounds are useful in therapy, especially adjunctive therapy, particularly they are indicated for use as: inhibitors of platelet activation, aggregation and degranulation, anti-thrombotic agents or in the treatment or prophylaxis of unstable angina, coronary angioplasty (PTCA), myocardial infarction, perithrombolysis, primary arterial thrombotic complications of atherosclerosis such as thrombotic or embolic stroke, peripheral vascular disease, myocardial infarction with or without thrombolysis, arterial complications due to interventions in atherosclerotic disease such as angioplasty, endarterectomy, stent placement, coronary and other vascular graft surgery, thrombotic complications of surgical or mechanical damage such as tissue salvage following accidental or surgical trauma, reconstructive surgery including skin and muscle flaps, conditions with a diffuse thrombotic/platelet consumption component such as disseminated intravascular coagulation, thrombotic thrombocytopaenic purpura, haemolytic uraemic syndrome, thrombotic complications of septicaemia, adult respiratory distress syndrome, anti-phospholipid syndrome, heparin-induced thrombocytopaenia and pre-eclampsia/eclampsia, or venous thrombosis such as deep vein thrombosis, venoocclusive disease, haematological conditions such as myeloproliferative disease, including thrombocythaemia, sickle cell disease; or in the prevention of mechanically-induced platelet activation *in vivo,* such as cardio-pulmonary bypass and extracorporeal membrane oxygenation (prevention of microthromboembolism), mechanically-induced platelet activation *in vitro,* such as use in the preservation of blood products, e.g. platelet concentrates, or shunt occlusion such as in renal dialysis and plasmapheresis, thrombosis secondary to vascular damage/inflammation such as vasculitis, arteritis, glomerulonephritis, inflammatory bowel disease and organ graft rejection, conditions such as migraine, Raynaud's phenomenon, atheromatous plaque formation/progression, vascular stenosis/restenosis and asthma, in which platelet-derived factors are implicated in the disease process.

According to the invention there is further provided the use of a compound according to the invention in the manufacture of a medicament for the treatment of the above disorders. The invention also provides a method of treatment of the above disorders which comprises administering to a patient suffering from such a disorder a therapeutically effective amount of a compound according to the invention.

The compounds may be administered topically, e.g. to the lung and/or the airways, in the form of solutions, suspensions, HFA aerosols and dry powder formulations; or systemically, e.g. by oral administration in the form of tablets, pills, capsules, syrups, powders or granules, or by parenteral administration in the form of sterile parenteral solutions or suspensions, by subcutaneous administration, or by rectal administration in the form of suppositories or transdermally.

The compounds of the invention may be administered on their own or as a pharmaceutical composition comprising the compound of the invention in combination with a pharmaceutically acceptable diluent, adjuvant or carrier. Particularly preferred are compositions not containing material capable of causing an adverse, e.g. an allergic, reaction.

Dry powder formulations and pressurised HFA aerosols of the compounds of the invention may be administered by oral or nasal inhalation. For inhalation the compound is desirably finely divided.

The compounds of the invention may also be administered by means of a dry powder inhaler. The inhaler may be a single or a multi dose inhaler, and may be a breath actuated dry powder inhaler.

One possibility is to mix the finely divided compound with a carrier substance, e.g. a mono-, di- or polysaccharide, a sugar alcohol or another polyol. Suitable carriers include sugars and starch. Alternatively the finely divided compound may be coated by another substance. The powder mixture may also be dispensed into hard gelatine capsules, each containing the desired dose of the active compound.

Another possibility is to process the finely divided powder into spheres which break up during the inhalation procedure. This spheronized powder may be filled into the drug reservoir of a multidose inhaler, e.g. that known as the Turbuhaler® in which a dosing unit meters the desired dose which is then inhaled by the patient. With this system the active compound with or without a carrier substance is delivered to the patient.

The pharmaceutical composition comprising the compound of the invention may conveniently be tablets, pills, capsules, syrups, powders or granules for oral administration; sterile parenteral or subcutaneous solutions, suspensions for parenteral administration or suppositories for rectal administration.

For oral administration the active compound may be admixed with an adjuvant or a carrier, e.g. lactose, saccharose, sorbitol, mannitol, starches such as potato starch, corn starch or amylopectin, cellulose derivatives, a binder such as gelatine or polyvinylpyrrolidone, and a lubricant such as magnesium stearate, calcium stearate, polyethylene glycol, waxes, paraffin, and the like, and then compressed into tablets. If coated tablets are required, the cores, prepared as described above, may be coated with a concentrated sugar solution which may contain e.g. gum arabic, gelatine, talcum, titanium dioxide, and the like. Alternatively, the tablet may be coated with a suitable polymer dissolved in either a readily volatile organic solvent or an aqueous solvent.

For the preparation of soft gelatine capsules, the compound may be admixed with e.g. a vegetable oil or polyethylene glycol. Hard gelatine capsules may contain granules of the compound using either the above mentioned excipients for tablets, e.g. lactose, saccharose, sorbitol , mannitol, starches, cellulose derivatives or gelatine. Also liquid or semisolid formulations of the drug may be filled into hard gelatine capsules.

Liquid preparations for oral application may be in the form of syrups or suspensions, for example solutions containing the compound, the balance being sugar and a mixture of ethanol, water, glycerol and propylene glycol. Optionally such liquid preparations may contain colouring agents, flavouring agents, saccharine and carboxymethylcellulose as a thickening agent or other excipients known to those skilled in art.

The invention is illustrated by the following examples. In the examples the NMR spectra were measured on a Varian Unity Inova 300 or 400 spectrometer and the MS spectra were measured as follows: EI spectra were obtained on a VG 70-250S or Finnigan Mat Incos-XL spectrometer, APCI spectra were obtained on Finnigan Mat SSQ7000 or a Micromass Platform spectrometer. Preparative HPLC separations were generally performed using a Novapak® , Bondapak® or Hypersil® column packed with BDSC-18 reverse phase silica. Flash chromatography (indicated in the Examples as (SiO₂)) was carried out using Fisher Matrix silica, 35-70 µm.

### Example 1

### [1S-[1α,2β,3β,4α (1S*,2R*)]]-N-Ethyl-2,3-dihydroxy-4-[7-[methyl(2-phenylcyclopropyl)amino]-5-(propylthio)-3H-[1,2,3]triazolo[4,5-d]pyrimidin-3-yl]cyclopentanecarboxamide

**a) [3a*R*-(3aα,4α,6α,6aα]-6-[[6-Chloro-5-nitro-2-(propylthio)-4-pyrimidinyl]amino]-*N*-ethyl-tetrahydro-2,2-dimethyl-4*H*-cyclopenta-1,3-dioxole-4-carboxamide**
   A solution of [3a*R*-(3aα,4α,6α,6aα)]-4-amino-*N*-ethyl-tetrahydro-2,2-dimethyl-4*H*-cyclopenta-1,3-dioxole-4-carboxamide (14.0g) (prepared as described in WO 9528160) in 1,4-dioxane (15ml) was added over 5 minutes to a stirred solution of 4,6-dichloro-5-nitro-2-(propylthio)pyrimidine (32.9g) (prepared as described in WO9703084) in 1,4-dioxane at 10°C. *N,N*-Diisopropylethylamine (16ml) was then added and the mixture allowed to warm to room temperature overnight. The reaction mixture was concentrated and the residue purified by chromatography (SiO₂, ether:isohexane 4:1 as eluant) to afford the subtitle compound (17.3g).
   MS (APCI) 460 (M+H⁺, 100%).
**b) [3a*R*-(3aα,4α,6α,6aα)]-6-[7-Chloro-5-(propylthio)-3*H*-[1,2,3]triazolo[4,5-*d*]pyrimidin-3-yl]-*N*-ethyl-tetrahydro-2,2-dimethyl-4*H*-cyclopenta-1,3-dioxole-4-carboxamide**
   To a solution of the product from step a) (32.0g) in acetic acid (300ml) was added iron powder (40.0g). The mixture was stirred at room temperature for 2 hours then filtered through Celite washing with ethyl acetate (3x150ml). The filtrate was concentrated to a volume of 150ml then cooled to 10°C. Sodium nitrite (7.5g) in water (25ml) was added and the solution allowed to warm to room temperature over 40 minutes. Water (200ml) was then added and the resultant precipitate collected by filtration, washing with water (100ml) and isohexane (100ml) afforded the subtitle compound (26.4g).
   MS (APCI) 441 (M+H⁺, 100%).
**c) [3a*R*-[3αa,4α,6α(1*R**,2*S**),6aα]]-*N*-Ethyl-tetrahydro-2,2-dimethyl-6-[7-[methyl(2-phenylcyclopropyl)amino]-5-(propylthio)-3*H*-[1,2,3]triazolo[4,5-*d*]pyrimidin-3-yl]-4*H*-cyclopenta-1,3-dioxole-4-carboxamide**
   A mixture of the product of step b) (800mg), *(1R-trans)-N-*methyl-2-phenylcyclopropanamine hydrochloride (prepared as described by C. Kaiser *et al, J*. Org. Chem., **1962**,*27*,768-773, using (1R-trans)-2-phenylcyclopropanamine, *[R-(R*^{***}*,R*^{***}*)]-2,3*-dihydroxybutanedioate (1:1), prepared as described byL.A. Mitscher *et al,* J. Med. Chem., **1986**,*29*, 2044) (366mg) and *N,N*-diisopropylethylamine (1.5ml) in dichloromethane (50ml) was stirred at room temperature for 16 hours. The reaction mixture was concentrated and the residue purified by chromatography (SiO₂, isohexane:ethylacetate 2:1 as eluant) to afford the subtitle compound (990mg).
   MS (APCI) 552 (M+H⁺, 100%).
**d) [1*S*-[1α,2β,3β,4α (1*S**,2*R**)]]-*N*-Ethyl-2,3-dihydroxy-4-[7-[methyl(2-phenylcyclopropyl)amino]-5-(propylthio)-3*H*-[1,2,3]triazolo[4,5-*d*]pyrimidin-3-yl]cyclopentanecarboxamide**
   A solution of the product from step c) (275mg) in trifluoroacetic acid (16ml) and water (4ml) was stirred at room temperature for 3 hours. The reaction mixture was concentrated, neutralised with aqueous sodium bicarbonate solution and then extracted with dichloromethane. The organic solution was dried, concentrated and the residue purified by chromatography (SiO₂, dichloromethane:methanol 97:3 as eluant) to afford the title compound (242mg).
   Mpt 154-157°C
   MS (APCI) 512 (M+H⁺, 100%).
   NMR δH (d₆-DMSO, 90°) 7.58 (1H, s), 7.32-7.17 (5H, m), 4.98 (1H, q), 4.81 (1H, d), 4.66 (1H, d), 4.46 (1H, q), 4.18 (1H, q), 3.16-2.95 (8H, m), 2.77-2.72 (1H, m), 2.41-2.23 (3H, m), 1.66-1.52 (3H, m), 1.47-1.43 (1H, m), 1.04 (3H, t), 0.94 (3H, t).

### Example 2

### [1S-[1α,2β,3β,4α(1S*,2R*)]]-N-Ethyl-2,3-dihydroxy-4-[7-[methyl(2-phenylcyclopropyl)amino]-5-(methylthio)-3H-[1,2,3]triazolo[4,5-d]pyrimidin-3-yl]cyclopentanecarboxamide

**a) [3a*R*-[3α,4α,6α(1*R**,2*S**),6α]]-*N*-Ethyl-tetrahydro-2,2-dimethyl-6-[7-[methyl(2-phenylcyclopropyl)amino]-5-(propylsulfonyl)-3*H*-[1,2,3]triazolo[4,5-*d*]pyrimidin-3-yl]-4*H*-cyclopenta-1,3-dioxole-4-carboxamide**
   3-Chloroperoxybenzoic acid (85%, 1.0g) was added to a suspension of the product of example 1, step c) (680mg) in ethanol (25ml) and the resulting solution stirred at room temperature for 24 hours. The reaction mixture was concentrated and the residue taken up in ethyl acetate (100ml), washed with 10% aqueous sodium metabisulfite solution (2 x 80ml) and 10% aqueous sodium bicarbonate solution (2x50ml) then dried and concentrated. The residue was purified by chromatography (SiO₂, ethyl acetate: isohexane 1:1 as eluant) to give the subtitle compound (780mg).
   MS (APCI) 584 (M+H⁺, 100%).
**b) [3a*R*-[3aα,4α,6α(1*R**,2*S**),6aα]-*N*-Ethyl-tetrahydro-2,2-dimethyl-6-[7-[methyl(2-phenylcyclopropyl)amino]-5-(methylthio)-3*H*-[1,2,3]triazolo[4,5-*d*]pyrimidin-3-yl-4*H*-cyclopenta-1,3-dioxole-4-carboxamide**
   Sodium thiomethoxide (300mg) was added to a stirred solution of the product from step a) (390mg) in dry tetrahydrofuran (20ml). The mixture was stirred at room temperature for 24 hours, concentrated and the residue purified by chromatography (SiO₂, isohexane:ethyl acetate 2:1 as eluent) to afford the subtitle compound (329mg).
   MS (APCI) 524 (M+H⁺, 100%).
**c) [1*S*-[1α,2β,3β,4α(1*S**,2*R**)]]-*N*-Ethyl-2,3-dihydroxy-4-[7-[methyl(2-phenylcyclopropyl)amino]-5-(methylthio)-3*H* [1,2,3]triazolo[4,5-*d*]pyrimidin-3-yl]-cyclopentanecarboxamide**
   The title compound was prepared according to the method of example 1, step d) using the product of step b).
   Mpt 152-153°C
   MS (APCI) 484 (M+H⁺, 100%).
   NMR δH (d₆-DMSO, 90°) 7.59 (1H, s), 7.32-7.17 (5H, m), 5.00 (1H, q), 4.83 (1H, d), 4.68 (1H, d), 4.46 (1H, q), 4.19 (1H, q), 3.57 (4H, s), 3.16-2.95 (2H, m), 2.80-2.73 (1H, m), 2.40 (3H, s), 2.36-2.20 (3H, m), 1.59-1.52 (1H, m), 1.47-1.41 (1H, m), 1.04 (3H, t).

### Example 3

### [1S-[1α,2β,3β,4α(1S*,2R*)]]-2,3-Dihydroxy-N-(2-hydroxyethyl)-4-[7-[methyl(2-phenylcyclopropyl)amino]-5-(propylthio)-3H-[1,2,3]triazolo[4,5-d]pyrimidin-3-yl]cyclopentanecarboxamide

**a) [3a*R*-(3aα,4α,6α,6aα)]-6-([5-Amino-6-chloro-2-(propylthio)-4- pyrimidinyl]amino]-tetrahydro-2,2-dimethyl-4*H*-cyclopenta-1,3-dioxole-4-carboxylic acid**
   Iron powder (10.0g) was added to a stirred solution of [3a*S*-(3aα,4β,7β,7aα)] 5-[6-chloro-5-nitro-2-(propylthio)pyrimidin-4-yl]-tetrahydro-2,2-dimethyl-4,7-methano-1,3-diox6lo[4,5-*c*]pyridin-6(3a*H*)-one (10.0g) (prepared as described in WO9703084) and calcium chloride (4.45g) in ethanol (140ml). The reaction mixture was heated at reflux for 10 minutes then filtered through Celite, washing with hot ethanol. The filtrate was concentrated to afford the subtitle compound (9.3g).
   MS (FAB) 405, 403 (M+H⁺), 405 (100%).
**b) [3a*R*-(3aα,4α,6α,6αa)]-6-[7-Chloro-5-(propylthio)-3*H*-[1,2,3]triazolo[4,5-d]pyrimidin-3-yl]-tetrahydro-2,2-dimethyl-4*H*-cyclopenta-1,3-dioxole-4-carboxylic acid**
   Isoamyl nitrite (6.02ml) was added to a solution of the product of step a) (9.28g) in acetonitrile (80ml) and the solution heated at 70°C for 1 hour. The cooled reaction mixture was concentrated and the residue purified by chromatography (SiO₂, ethyl acetate:isohexane 2:1 as eluant) to afford the subtitle compound (7.9g).
   MS (FAB) 416, 414 (M+H⁺), 414 (100%).
**c) [3a*R*-[3aα,4α,6α(1*R**,2*S**),6aα]]-Tetrahydro-2,2-dimethyl-6-[7-[2-phenylcyclopropyl)methylamino]-5-(propylthio)-3*H*-[1,2,3]triazolo[4,5-*d*]pyrimidin-3-yl]-4*H*-cyclopenta-1,3-dioxole-4-carboxylic acid**
   The sub-title compound was prepared according to the method of example I, step c) using the product of step b).
   MS (APCI) 525 M+H⁺, 100%).
**d) [3a*R*-[3aα,4α,6α(1*R**,2*S**),6aα]]-Tetrahydro-*N*-(2-hydroxyethyl)-2,2-dimethyl-6-[7-[methyl(2-phenylcyclopropyl)amino]-5-(propylthio)-3*H*-[1,2,3]triazolo[4,5-d]pyrimidin-3-yl]-4*H*-cyclopenta-1,3-dioxole-4-carboxamide**
   Ethanolamine (0.06ml) was added to a solution of *N,N*-diisopropylethylamine (0.2ml), bromo-tris-pyrrolidino-phosphonium hexafluorophosphate (224mg) and the product of step c) (210mg) in dichloromethane (20ml). The reaction mixture was stirred at room temperature for 4 hours then concentrated. The residue was purified by chromatography (SiO₂, dichloromethane:methanol 98:2 as eluant) to afford the subtitle compound (180mg).
   MS (APCI) 568 (M+H⁺, 100%).
**e) [1*S*-[1α,2β,3β,4α(1*S**,2*R**)]]-2,3-Dihydroxy-*N*-(2-hydroxyethyl)-4-[7-[methyl(2-phenylcyclopropyl)amino]-5-(propylthio)-3*H*-[1,2,3]triazolo[4,5-d]pyrimin-3-yl]cyclopentanecarboxamide**
   A solution of the product from step d) (470mg) in 1,4-dioxane (5ml) and 2M aqueous hydrochloric acid (5ml) was stirred at room temperature for 16 hours. The reaction mixture was concentrated and the residue taken into ethyl acetate (50ml) and washed with water (2 x 50ml). The organic phase was dried and concentrated and the residue purified by chromatography (SiO₂, dichoromethane:methanol 97:3 as eluent) to afford the title compound (90 mg).
   MS (APCI) 568 (M+H⁺, 100%).
   NMR δH (d₆-DMSO, 90°) 7.59 (1H, t), 7.30-7.18 (5H, m), 4.90 (1H, m), 4.82 (1H, d), 4.68 (1H, d), 4.47 (1H, m), 4.35 (1H, m), 4.19 (1H, m), 3.59 (3H, s), 3.42 (2H, m), 3.20 (2H, m), 3.10-2.90 (2H, m), 2.80 (1H, m), 2.40-2.20 (3H, m), 1.64-1.50 (3H, m), 1.42 (1H, m), 0.93 (3H, t).

### Example 4

### [1S-[1α,2β,3β,4α (1S*,2R*)]]-4-[7-[[2-(3,4-difluorophenyl)cyclopropyl]methylaminol]-5-(propylthio)-3H-[1,2,3]triazolo[4,5-d]pyrimidin-3-yl]-N-ethyl 2,3-dihydroxycyclopentanecarboxamide

**a) [3a*R*-[3aα,4α,6α(1*R**,2*S**),6aα]]-6-[7-[2-(3,4-Difluorophenyl)cyclopropylamino]-5-(propylthio)-3*H*-[1,2,3]triazolo[4,5-*d*]pyrimidin-3-yl]-tetrahydro-2,2-dimethyl 4*H*-cyclopenta-1,3-dioxole-4-carboxamide**
   A mixture of the products of Example 1, step b) (299mg), *(1R-trans)-2-(3,4*-difluorophenyl)-N-methylcyclopropanamine, [*R*-(*R**,*R**)]-2,3-dihydroxybutanedioate (1:1) (prepared as described in WO9905143) (238mg) and *N,N*-diisopropylethylamine (0.47ml) in dichloromethane (8ml) were stirred at room temperature for 16 hours. The reaction mixture was dried and concentrated to afford the subtitle compound (330mg).
   MS (APCI) 574 (M+H⁺, 100%).
**b) 1*S*-1α,2β,3β,4α(1*S**,2*R**)]1-4-[7-[[2-(3,4-difluorophenyl)cyclopropyl]methylamino]-5-(propylthio)-3*H*-[1,2-3]triazolo[4,5-*d*]pyrimidin-3-yl]-*N*-ethyl 2,3-dihydroxycyclopentanecarboxamide**
   A mixture of the product from step a) (180mg), methyl iodide (59µl) and potassium carbonate (130mg) was stirred together in *N,N* dimethylformamide (2ml) at room temperature for 5 hours. Aqueous ammonium chloride was added and the reaction mixture extracted with ethyl acetate. The organic extracts were washed with water, dried and concentrated. The resulting pale yellow solid was treated with TFA (3ml) in water (3ml) and stirred at room temperature for 2 hours. Aqueous sodium bicarbonate was added and the reaction mixture extracted with ethyl acetate. The organic extracts were dried and concentrated to afford the title compound (103mg).
   Mpt 183-185°C
   MS (APCI) 548 (M+H⁺, 100%).
   NMR δH (d₆-DMSO) 7.61 (1H, s), 7.33-7.26 (2H, m), 7.10-7.07 (1H, m), 4.96-4.92 (1H, m), 4.83-4.82 (1H, m), 4.70-4.68 (1H, m), 4.48-4.20 (1H, m), 4.19-4.16 (1H, m), 3.55 (4H, s), 3.08-3.04 (4H, m), 3.01-2.93 (1H, m), 2.79-2.73 (1H, m), 2.40-2.21 (3H, m), 1.68-1.57 (3H, m), 1.49-1.44 (1H, m), 1.08 (3H, t), 0.94 (3H, t).

### Example 5

### [1S-[1α,2β,3β,4α (1S*,2R*)]]-4-[7-[[2-(4-Chlorophenyl)cyclopropyl]methylamino]-5-(propylthio)-3H-[1,2,3]triazolo[4,5-d]pyrimidin-3-yl]-N-ethyl-2,3-dihydroxycyclopentanecarboxamide

**a) (1*R-trans*)-*N*-[2-(4-Chlorophenyl)cyclopropyl]acetamide**
   Acetic anhydride (0.5ml) was added to a solution of (1*R-trans*)-2-(4-chlorophenyl)cyclopropanamine (0.72g), (prepared as described in WO9905143) in 1M sodium hydroxide (5ml) and stirred for 1 hour. The mixture was extracted with dichloromethane and ethyl acetate (3 times each) and the combined organic extracts were dried and evaporated to afford the subtitle compound (0.92g)
   MS (APCI) 210,212 (M+H⁺) 210 (100%).
**b) (1*R*-trans)-*N*-[2-(4-Chlorophenyl)cyclopropyl]-*N*-methylacetamide**
   Sodium hydride (228mg, 60% dispersion in oil) was added to a solution of the product from step a) (0.92g) and methyl iodide (0.4ml) in THF (6ml) and the mixture was stirred for 18 hours. Aqueous ammonium chloride was added and the mixture extracted with diethyl ether (3 times). The combined organic extracts were dried and evaporated. The crude product was purified by chromatography (SiO₂, isohexane:ethyl acetate 1:3 as eluent) to afford the subtitle compound (1.78g).
   MS (APCI) 224,226 (M+H⁺) 224 (100%).
**c) (1*R-trans*)-2-(4-chlorophenyl)-*N*-methylcyclopropanamine, hydrochloride**
   A solution of the product from step b) (914mg) in 4M hydrochloric acid (4ml) was heated under reflux for 8 hours. The solvent was removed *in vacuo* and the residue was azeotroped with toluene and then triturated with acetone-isohexane to afford the subtitle compound (850mg)
   Mpt 150-1°C
   MS (APCI) 182 (M-Cl⁻, 100%).
**d) [1*S*-[1α,2β,3β,4α(1*S**,2*R**)]]-4-[7-[[2-(4-Chlorophenyl)cyclopropyl]methylamino]-5-(propylthio)-3*H*-[1,2,3]triazolo[4,5-*d*]pyrimidin-3-yl]-*N*-ethyl-2,3-dihydroxycyclopentanecarboxamide**
   A mixture of the products of step c) (120mg), Example 1, step b) (220mg) and *N,N*-diisopropylethylamine (0.35ml) in dichloromethane (8ml) was stirred at room temperature for 16 hours. The reaction mixture was concentrated and the residue treated with trifluoroacetic acid (3ml) in water (3ml). The reaction mixture was stirred at room temperature for I hour, neutralised with aqueous sodium bicarbonate solution and extracted with ethyl acetate. The organic extracts were dried, concentrated and the crude product recrystallised (acetonitrile) to afford the title compound (129mg).
   Mpt 166-168°C
   MS (APCI) 546, 548 (M+H⁺), 546 (100%).
   NMR δH (d₆-DMSO) 7.93 (1H, m), 7.37-7.35 (2H, m), 7.28-7.25 (2H, m), 5.12-5.10 (1H, m), 4.99-4.93 (2H, m), 4.47-4.40 (1H, m), 4.12-4.09 (1H, m), 3.33 (1H, s), 3.12-3.05 (3H, m), 2.76-2.70 (1H, m), 2.33-2.20 (3H, m), 1.65-1.48 (4H, m), 1.03 (3H, t), 0.95 (3H, t).

### Example 6

### [1S-[1α,2β,3β,4α(1S*,2R*)]]-4-(7-[[2-(4-Aminosulfonylphenyl)cyclopropyl]methylamino]-5-(propylthio)-3H-[1,2,3]triaxolo[4,5-d]pyrimidin-3-yl]-N-ethyl-2,3-dihydroxycyclopentanecarboxamide

**a) (1*R-trans)-N* 2-Phenylcyclopropylacetamide**
   The subtitle compound was prepared as described for Example 6, step a) using *(1R-trans)*-2-phenylcyclopropanamine, [*R*-(*R**,*R**)]-2,3-dihydroxybutanedioate (1:1) (prepared as described by L.A. Mitscher *et al,* J. Med. Chem., **1986**,*29*, 2044).
   MS (APCI) 176 (M+H⁺, 100%)
**b) (1*R-trans*)-*N*-Methyl-*N*-2-phenylcyclopropylacetamide**
   The subtitle compound was prepared as described for Example 6, step b) using the product of step a).
   MS (APCI) 190 (M+H⁺, 100%)
**c) (1*S-trans*)-4-[2-(N-methylacetylamino)cyclopropyl]benzenesulfonylchloride**
   Chlorosulfonic acid (8ml) was slowly added to the product from step b) (2.17g) at -78°C and then allowed to warm to 20°C and stirred for 20 hours. The mixture was poured into ice-water (100ml) and extracted with ethyl acetate (3 times). The combined organic extracts were dried and evaporated to afford the subtitle compound (3.48g).
   MS (APCI) 288, 290 (M+H⁺), 288 (100%)
**d) (1*S-trans*)-*N*-[2-(4-Aminosulfonylphenyl)cyclopropyl]-*N*-methylacetamide**
   A mixture of the product from step c) (1.74g) in conc. aqueous ammonia (5ml) was heated at 50°C for 1.5 hours. The mixture was extracted with ethyl acetate (8 times) and the combined organic layers were dried and evaporated. The crude product purified by chromatography (SiO₂, dichloromethane:methanol 14:1 as eluent) to afford the subtitle compound (1.30g).
   NMR δH (d₆-DMSO) 7.73 (2H, d), 7.32 (2H, d), 7.25 (2H, s), 3.03-2.98 (1H, m), 2.84 (3H, s), 2.44-2.37 (1H, m), 2.00 (3H, s), 1.56-1.41 (2H, m).
**e) (1*S-trans*)-4-[2-(Methylamino)cyclopropyl]benzenesulfonamide, hydrochloride**
   A solution of the product from step d) (1.19g) in 4M HCl (7ml) was heated under reflux for 6 hours. After cooling the precipitate was collected, washed with ether and dried to afford the subtitle compound (782mg).
   Mpt 173-4°C.
   MS (APCI) 227 (M-Cl⁻, 100%).
**f) [1*S*-[1α,2β,3β,4α(1*S**,2*R**)]]-4-[7-[[2-(4-Aminosulfonylphenyl)cyclopropyl]methylamino]-5-(propylthio)-3*H*-[1,2,3]triazolo[4,5-*d*]pyrimidin-3-yl]-N-ethyl-2,3-dihydroxycyclopentanecarboxamide**
   A mixture of the product from step e) (144mg), the product from Example 1 step b) (219mg) and *N,N*-diisopropylethylamine (0.21ml) in THF (5ml) was stirred at room temperature for 16 hours. Aqueous sodium dihydrogen phosphate solution was added, the mixture was extracted with ethyl acetate (3 times). The combined organic layers were dried, evaporated and the residue dissolved in acetic acid (4ml) and water (1ml) and heated at 80°C for 3 hours. The solvent was removed in vacuo, aqueous ammonia added and reevaporated. The residue was purified by chromatography (SiO₂, dichloromethane:methanol 11:1 as eluent) and the crude product triturated with ether to afford the title compound (224mg).
   Mpt 132-5°C.
   MS (APCI) 591 (M+H⁺, 100%).
   NMR δH (d₆-DMSO) 7.92 (1H, t), 7.75 (2H, d), 7.42 (2H, d), 7.31 (2H, s), 4.98 (1H, q), 4.46-4.41 (1H, m), 4.11 (1H, t), 4.00-2.90 (9H, m), 3.09 (2H, quintet), 2.72 (1H, dq), 2.38-2.18 (2H, m), 1.80-1.44 (4H, m), 1.02 (3H, t), 0.91 (3H, s).

### Example 7

### [1S-[1α,2β,3β,4α(1S*,2R*)]]-N-Ethyl-2,3 dihydroxy4-[7-[methyl[2-(4-methytaminosulfonylphenyl)cyclopropyl]amino]-5-(propylthio)-3H-[1,2,3]triazolo[4,5-d]pyrimidin-3-yl]cyclopentanecarboxamide

**a) (1*S-trans*)-*N*-Methyl-*N*-[2-(4-methylaminosulfonylphenyl)cyclopropyl]acetamide**
   A mixture of the product from Example 7, step c) (1.74g) in 40% aqueous methylamine (6ml) was heated at 50°C for 1.5 hours. The mixture was extracted with ethyl acetate (7 times). The combined organic extracts were dried and evaporated and the crude product purified by chromatography (SiO₂, dichloromethane:methanol 11:1 as eluent) to afford the subtitle compound (1.40g).
   NMR δH (d₆-DMSO) 7.67 (2H, d), 7.37 (2H, d), 7.31 (1H, s), 3.07-2.99 (1H, m), 2.83 (3H, s), 2.43-2.38 (4H, m), 2.00 (3H, s), 1.58-1.41 (2H, m).
**b) (1*S-trans*)-*N*-Methyl-4-[2-(methylamino)cyclopropyl]benzenesulfonamide hydrochloride**
   A solution of the product from step a) (1.37g) in 4M HCl (7ml) was heated under reflux for 8h. After cooling the solvent was removed *in vacuo* and the residue crystallised (isopropanol) to afford the subtitle compound (1.01g).
   Mpt 174-5°C.
   MS (APCI) 241 (M-Cl⁻, 100%).
**c) [1*S*-[1α,2β,3β,4α(1*S**,2*R**)]]-*N*-Ethyl-2,3-dihydroxy-4-[7-[methyl[2-(4-methylaminosulfonylphenyl)cyclopropyl]amino]-5-(propylthio)-3*H*-[1,2,3]triazolo[4,5,*d*]pyrimidin-3-yl]cyclopentanecarboxamide**
   The title compound was prepared according to the method of Example 7, step f) using the product from step b) and the product from Example 1, step b).
   Mpt 124-6°C.
   MS (APCI) 605 (M+H⁺, 100%).
   NMR δH (d₆-DMSO) 7.91 (1H, t), 7.70 (2H, d), 7.45 (2H, d), 7.39 (1H, q), 5.05 (1H. s), 4.97 (1H, q), 4.44 (1H, m), 4.11 (1H, q), 4.00-2.85 (7H, m), 3.12 (2H, quintet), 2.72 (1H. dt), 2.43-2.18 (3H, m), 2.41 (3H, d), 1.80-1.43 (4H, m), 1.03 (3H, t), 0.87 (3H, s).

### Example 8

### [1S-[1α,2β,3β,4α (1S*,2R*)]]-N-(2-Fluoroethyl)-2,3-dihydroxy-4-[7-[methyl(2-phenylcyclopropyl)amino]-5-(propylthio)-3H-[1,2,3]triazolo(4,5-d]pyrimidin-3-yl]cyclopentanecarboxamide

2-Fluoroethylamine hydrochloride (23mg) was added to a solution of *N,N*-diisopropylethylamine (110µl), 1-hydroxybenzotriazole hydrate (39mg), 2-(1*H*-benzotriole-1-yl)-1,1,3,3-tetramethyluroniumtetrafluoroborate (81mg) and the product of Example 3, step c) (110mg) in *N,N*-dimethylformamide (10ml). The reaction mixture was stirred at room temperature for 1.5 hours, poured into 2N HCl and the resulting precipitate washed with water and dried to afford a white solid. Trifluoroacetic acid (3ml) in water (2ml) were added to this solid in methanol (2ml) and the reaction mixture stirred at room temperature for 2 hours. The reaction mixture was basified with 2N potassium carbonate solution and extracted with ethyl acetate. The organic extracts were washed with water, dried and concentrated and the crude product purified by chromatography (SiO₂, dichloromethane:methanol 98:2 as eluent) to afford the title compound (74mg).
Mpt 141-143°C
MS (APCI) 530 (M+H⁺, 100%).

NMR δH(d₆-DMSO) 8.20-8.18 (1H, m), 7.34-7.20 (5H, m), 5.14-5.12 (1H, m), 5.02-4.98 (2H, m), 4.54-4.50 (1H, m), 4.56-4.43 (1H, m), 4.38-4.34 (1H, m), 4.14-4.13 (1H, m), 3.45-3.42 (2H, m), 3.19-2.89 (4H, m), 2.83-2.81 (2H, m), 2.35-2.25 (2H, m), 1.59-1.54 (3H, m), 0.93 (3H, t).

### BIOLOGICAL ASSAYS

### Washed Platelet Preparation

Human venous blood (100 ml), obtained from a healthy volunteer, was divided equally between 3 tubes, each containing 3.2% trisodium citrate (4 ml) as an anti-coagulant. The tubes were centrifuged for 15 minutes at 240g to obtain platelet-rich plasma (PRP) to which prostacyclin (PGI₂ 300 ng·ml⁻¹) was added to stabilize platelets during the washing procedure. Red cell-free PRP was obtained by centrifugation for 10 minutes at 125g and, following further centrifugation for 15 minutes at 640g, the supernatant was discarded and the platelet pellet in each tube resuspended in modified, calcium-free, Tyrodes (CFT) solution (10 ml, composition: NaCI, 137.0 mM; NaHCO₃, 11.9 mM; Na₂PO₄, 0.4 mM; KCl, 2.7 mM; MgCl₂, 1.1 mM and dextrose, 5.55 mM), gassed with 95% O₂/5% CO₂ and maintained at 37°C. Following addition of PGI₂ (300 ng·ml⁻¹), the pooled suspension was centrifuged once more for 15 minutes at 640g. The supernatant was discarded and the platelets resuspended in CFT to give a final platelet count of 200-250 x 10³·µl⁻¹. The platelets were used within 30 minutes for radioligand binding studies.

### Binding Assays

Binding assays were performed in 96-well plates, with each well containing aliquots of CFT (250 µl) consisting of 0.1 µCi [¹²⁵I]-[1*S*-[1α,2β,3β,4β(*E*)]]-2,3-dihydroxy-4-[7-(3-iodo-prop-2-enylamino)-5-(propylthio)-3*H*-[ 1,2,3]triazolo[4,5-d]pyrimidin-3-yl]cyclopentanecarboxylic acid (50 µl, final concentration 0.18 nM) and washed platelets at a concentration of 200-250 x 10³·µl⁻¹(200 µl, final concentration 160-200 x 10³·µl⁻¹. The plates were incubated for 30 minutes at room temperature on a plate shaker (Stuart scientific; model SO1, setting 6) prior to terminating the reactions by filtration. Filtration was performed using either a MACHIII cell harvester with 2 x 2 second wash periods (with CFT) on to Whatman GF/B filter plates or a Wallac cell harvester using glass fibre printed filtermats type A, with a 7 second wash time (with CFT). The resultant filterplates were then, in the case of the MACHIII, sealed and Microscint 20 (50 ml) added prior to determination of [¹²⁵I] levels by scintillation counting on a Packard Topcounter or, in the case of the Wallac filtermats, the individual wells were punched into vials for determination of [¹²⁵I] in a Riastar gamma counter.

Non-specific binding was determined in the presence of the standard P2Y_{ADP} antagonist, 2-propylthio-D-β,γ-dichloromethylene ATP (10 µM), as described by Humphries et al., Br. J. Pharmacology (**1995**), *115,* 1110-1116. All compounds were tested in duplicate over the appropriate concentration range with solvent controls in parallel.

### Data Analysis

Results were expressed as specific binding in CPM and were calculated by subtracting the non-specific binding from the total binding achieved at each concentration. For each compound, a binding affinity (IC₅₀) was calculated by linear interpolation of the concentration/inhibition curve, using the software package Excel. The IC₅₀ value being the concentration at which a 50% reduction in specific binding of [¹²⁵I]-[1*S*-[1α,2β,3β,4α(*E*)]]-2,3-dihydroxy-4-[7-(3-iodo-prop-2-enylamino-5-(propylthio)-3*H*-[1,2,3]triazolo[4,5-*d*]pyrimidin-3-yl]cyclopentanecarboxylic acid was achieved. Results were reported as pKi values which are equal to the negative logarithm of the IC₅₀ (pIC₅ₒ) in this system (Cheng Prusoff).

## Claims

1. A compound of formula (I) 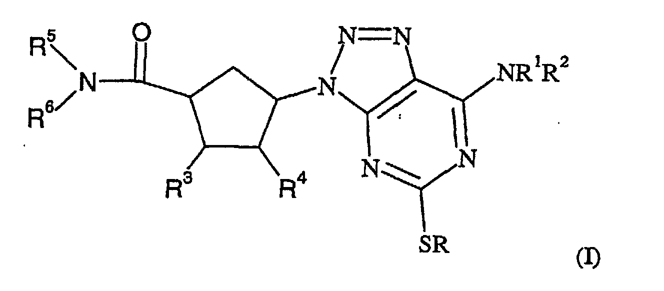 wherein:
R is C₁₋₆ alkyl optionally substituted by one or more halogen atoms;
R¹ is C₁₋₄ alkyl;
R² is C₁₋₆-alkyl or a C₃₋₈-cycloalkyl group optionally substituted by phenyl optionally substituted by one or more substituents selected from halogen, SO₂NR⁸R⁹, and C₁₋₆ alkyl which is optionally substituted by one or more halogen atoms;
R³ and R⁴ are both hydroxy;
R⁵ is hydrogen or C₁₋₆ alkyl, and R⁶ is C₁₋₆ alkyl optionally substituted by C₃₋₆-cycloalkyl or R⁶ is C₃₋₆-cycloalkyl or R⁵ and R⁶ together with the nitrogen atom to which they are attached form a 5- to 7-membered saturated ring;
R⁸, R⁹ are independently hydrogen or C₁₋₆ alkyl; or a pharmaceutically acceptable salt or solvate thereof.

2. A compound according to claim 1 in which R is methyl or propyl.

3. A compound according to claim 1 or 2 in which R¹ is methyl.

4. A compound according to any one of claims 1 to 3 in which R² is cyclopropyl optionally substituted by phenyl, 4-chlorophenyl, 3,4-difluorophenyl, 4-(aminosulphonyl)phenyl or 4-(methylaminosulphonyl)phenyl.

5. A compound according to any one of claims 1 to 4 in which R⁵ is hydrogen and R⁶ is C₁₋₆ alkyl optionally substituted by hydroxy or fluorine.

6. A compound according to any one of claims 1 to 5 with the following stereochemistry

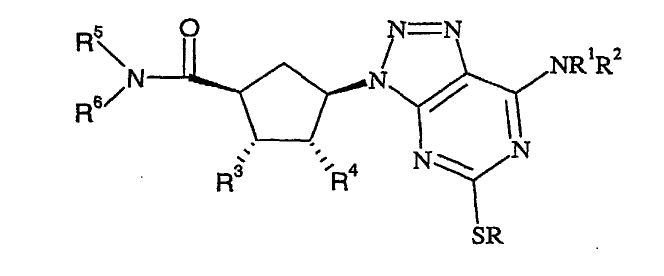

7. A compound according to claims 1 to 6 which is:
[1*S* [1α,2β,3β,4α(1*S**,2*R**)]]-*N*-Ethyl-2,3-dihydroxy-4-[7-[methyl(2-phenylcyclopropyl)amino]-5-(propylthio)-3*H*-[1,2,3]triazolo[4,5-*d*]pyrimidin-3- yl]cyclopentanecarboxamide,
[1*S*-[1α,2β,3β,4α(1*S**,2*R**)]]-*N*-Ethyl-2,3-dihydroxy-4-[7-[methyl(2-phenylcyclopropyl)amino]-5-(methylthio)-3*H*-[1,2,3]triazolo[4,5-*d*]pyrimidin-3-yl]cyclopentanecarboxamide,
[1*S*-[1α,2β,3β,4α(1*S**,2*R**)]]-2,3-Dihydroxy-*N*-(2-hydroxyethyl)-4-[7-[methyl(2-phenylcyclopropyl)amino]-5-(propylthio)-3*H* [1,2,3]triazolo[4,5-*d*]pyrimidin-3-yl]cyclopentanecarboxamide,
[1*S*-[1α,2β,3β,4α(1*S**,2*R**)]]-2,3-Dihydroxy-4-[7-[methyl(2-phenylcyclopropyl)amino]-5-(propylthio)-3*H*-[1,2,3]triazolo [4,5-*d*]pyrimidin-3-yl]cyclopentanecarboxamide,
[1*S*-[1α,2β,3β,4α(1*S**,2*R**)]]-4-[7-[[2-(3,4-difluorophenyl)cyclopropyl]methylamino]-5-(propylthio)-3*H* [1,2,3]triazolo[4,5-*d*]pyrimidin-3-yl]-*N*-ethyl 2,3-dihydroxycyclopentanecarboxamide,
[ 1*S*-[1α,2β,3β,4α(1*S**2*R**)]]-4-[7-[[2-(4-Chlorophenyl]cyclopropyl]methylamino]-5-(propylthio)-3*H*-[1,2,3]triazolo[4,5-*d*]pyrimidin-3-yl]-*N*-ethyl-2,3-dihydroxycyclopentanecarboxamide,
[ 1*S*-[1α,2β,3β,4α( 1*S**,2*R**)]]-4-[7-[[2-(4-Aminosulfonylphenyl)cyclopropyl]methylamino]-5-(propylthio)-3*H*-[1,2,3]triazolo[4,5-*d*]pyrimidin-3-yl]-*N*-ethyl-2,3-dihydroxycyclopentanecarboxamide,
[1*S*-[1α,2β,3β,4α(1*S**,2*R**)]]-*N*-Ethyl-2,3-dihydroxy-4-[7-[methyl[2-(4-methylaminosulfonylphenyl)cyclopropyl] amino]-5-(propylthio)-3*H*-[1,2,3]triazolo[4,5-*d*]pyrimidin-3-yl]cyclopentanecarboxamide,
[1*S*-[1α,2β,3β,4α(1*S**,2*R**)]]-*N*-(2-Fluoroethyl)-2,3-dihydroxy-4-[7-[methyl(2-phenylcyclopropyl)amino]-5-(propylthio)-3*H* [1,2,3]triazolo[4,5-*d*]pyrimidin-3-yl]cyclopentanecarboxamide or pharmaceutically acceptable salts or solvates thereof.

8. A pharmaceutical composition comprising a compound according to any one of claims 1 to 7 in combination with a pharmaceutically acceptable diluent, adjuvent or carrier.

9. A compound according to any one of claims 1 to 8 for use in therapy.

10. A process for the preparation of a compound of formula (I) which comprises;
(a) reaction of a compound of formula (II): 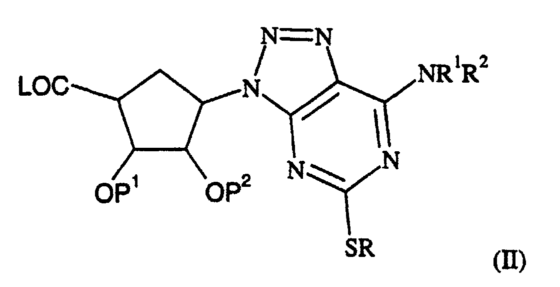 where R, R¹ and R² are as defined in formula (I) or are protected derivatives thereof, P¹ and P² are hydrogen or protecting groups and L is a leaving group, with a compound of formula (III):
R⁵R⁶NH (III)
where R¹ and R² are as defined in formula (I), or
(b) reacting a compound of formula (IV): 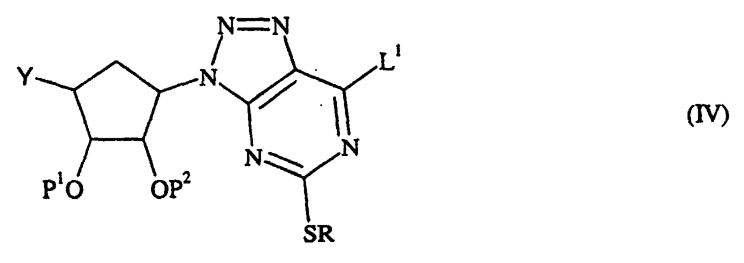
wherein Y is CO₂H, C0₂ R' or CONR⁵R⁶ and R, R⁵, R⁶, P¹ and P² are as defined above and L¹ is a leaving group and R' is a C₁₋₆-alkyl or benzyl group, with an amine NHR¹R² or a salt of NHR¹R² wherein R² is as defined above and optionally thereafter (a) or (b) and in any order:
• converting one or more functional groups into a further functional groups
• removing any protecting groups
• forming a pharmaceutically acceptable salt or solvate.

## Patentansprüche

1. Verbindungen der Formel (I) 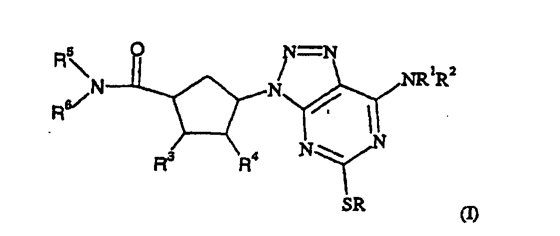 in welcher:
R für gegebenenfalls durch ein oder mehrere Halogenatome substituiertes C₁₋₆-Alkyl steht;
R¹ für C₁₋₄-Alkyl steht;
R² für C₁₋₆-Alkyl oder eine C₃₋₈-Cycloalkylgruppe:, gegebenenfalls substituiert durch Phenyl, das gegebenenfalls durch einen oder mehrere Substituenten ausgewählt aus Halogen, SO₂NR⁸R⁹ und gegebenenfalls durch ein oder mehrere Halogenatome substituiertes C₁₋₆-Alkyl substituiert ist, steht;
R³ und R⁴ beide für Hydroxy stehen;
R⁵ für Wasserstoff oder C₁₋₆-Alkyl steht, und R⁶ für gegebenenfalls durch C₃₋₆-Cycloalkyl substituiertes C₁₋₆-Alkyl steht, oder R⁶ für C₃₋₆-Cycloalkyl steht, oder R⁵ und R⁶ zusammen mit dem Stickstoffatom, an das sie gebunden sind, einen 5- bis 7gliedrigen gesättigten Ring bilden;
R⁸ und R⁹ unabhängig voneinander für Wasserstoff oder C₁₋₆-Alkyl stehen; und deren pharmazeutisch unbedenkliche Salze und Solvate.

2. Verbindungen nach Anspruch 1, wobei R für Methyl oder Propyl steht.

3. Verbindungen nach Anspruch 1 oder 2, wobei R¹ für Methyl steht.

4. Verbindungen nach einem der Ansprüche 1 bis 3, wobei R² für Cyclopropyl steht, das gegebenenfalls durch Phenyl, 4-Chlorphenyl, 3,4-Difluorphenyl, 4-(Aminosulfonyl)phenyl oder 4-(Methylaminosulfonyl)phenyl substituiert ist.

5. Verbindungen nach einem der Ansprüche 1 bis 4, wobei R⁵ für Wasserstoff und R⁶ für gegebenenfalls durch Hydroxy oder Fluor substituiertes C₁₋₆-Alkyl steht.

6. Verbindungen nach einem der Ansprüche 1 bis 5 mit der folgenden Stereochemie

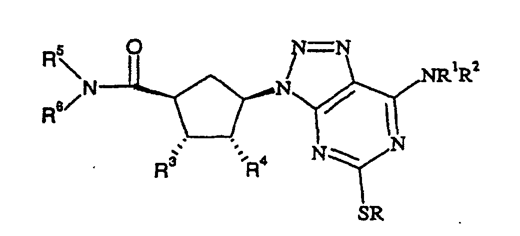

7. Verbindungen nach einem der Ansprüche 1 bis 6, bei denen es sich um:
[1*S*-(1α,2β,3β,4α(1*S**,2*R**)]]-*N*-Ethyl-2,3-dihydroxy-4-[7-[methyl(2-phenylcyclopropyl)amino]-5-(propylthio)-3*H*-[1,-2,3]triazolo[4,5-*d*]pyrimidin-3-yl]-cyclopentancarbonsäureamid,
[1*S*-[1α,2β,3β,4α(1*S**,2*R**)]]-*N*-Ethyl-2,3-dihydroxy-4-[7-[methyl(2-phenylcyclopropyl)amino]-5-(methylthio)-3*H*-[1,2,3] triazolo[4,5-d]pyrimidin-3-yl]-cyclopentancarbonsäureamid,
[1*S*-[1α,2β,3β,4α(1*S**,2*R**)]]-2,3-Dihydroxy-*N*-(2-hydroxyethyl)-4-[7-[methyl(2-phenylcyclopropyl)-amino]-5-(propylthio)-3*H*-[1,2,3]triazolo[4,5-*d*]-pyrimidin-3-yl]cyclopentancarbonsäureamid,
[1*S*-[1α,2β,3β,4α(1*S**,2*R**)]]-2,3-Dihydroxy-4-[7-[methyl(2-phenylcyclopropyl)amino]-5-(propylthio)-3*H*-[1,2,3]triazolo[4,5-*d*]pyrimidin-3-yl]cyclopentancarbonsäureamid,
[1*S*-[1α,2β,3β,4α(1*S**,2*R**)]]-4-[7-[[2-(3,4Difluorphenyl]cyclopropyl]methylamino]-5-(propylthio)-3*H*-[1,2,3)triazolo[4,5-*d*]pyrirnidin-3-yl]-*N*-ethyl-2,3-dihydroxycyclopentancarbonsäureamid,
[1*S*-[1α,2β,3β,4α(1*S**,2*R**)]]-4-[7- [[2-(4-Chlorphenyl)cyclopropyl]methylamino]-5-(propylthio)-3*H*-[1,2,3]triazolo[4,5-*d*]pyrimidin-3-yl] -*N*-ethyl-2,3-dihydroxycyclopentancarbonsäureamid,
[1*S*-[1α,2β,3β,4α(1*S**,2*R**)]]-4-[7-[[2-(4-Amino-sulfonylphenyl)cyclopropyl]methylamino]-5-(propylthio)-3*H*-[1,2,3]triazolo[4,5-*d*]pyrimidin-3-yl]-*N*-ethyl-2,3-dihydroxycyclopentancarbonsäureamid,
[1*S*-[1α,2β,3β,4α(1*S**,2*R**)]]-*N*-Ethyl-2,3-dihydroxy-4-[7-[methyl[2-(4-methylaminosulfonylphenyl)-cyclopropyl]amino]-5-(propylthio)-3*H*-[1,2,3]-triazolo[4,5-*d*]pyrimidin-3-yl]cyclopentancarbonsäureamid
[1*S*-[1α,2β,3β,4α(1*S**,2*R**)]]-*N*-(2-Fluorethyl)-2,3-dihydroxy-4-[7-[methyl [2-phenylcyclopropyl)amino]-5-(propylthio)-3*H*-[1,2,3] triazolo [4, 5-d]pyrimidin-3-yl]cyclopentancarbonsäureamid und pharmazeutisch unbedenkliche Salze und Solvate davon handelt.

8. Pharmazeutische Zusammensetzung, enthaltend eine Verbindung nach einem der Ansprüche 1 bis 7 in Kombination mit einem pharmazeutisch unbedenklichen Verdünnungsmittel, Adjuvans oder Träger.

9. Verbindungen nach einem der Ansprüche 1 bis 8 zur Verwendung in der Therapie.

10. Verfahren zur Herstellung einer Verbindung der Formel (I), bei dem man
(a) eine Verbindung der Formel (II): 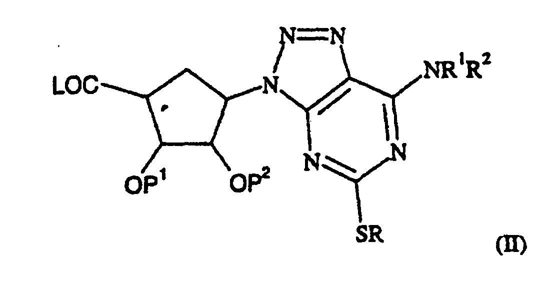 wobei R, R¹ und R² wie in Formel (I) definiert sind oder geschützte Derivate davon sind, P¹ und P² für Wasserstoff oder Schutzgruppen stehen und L für eine Abgangsgruppe steht, mit einer Verbindung der Formel (III):
R⁵R⁶NH (III)
wobei R¹ und R² wie in Formel (I) definiert sind, umsetzt, oder
(b) eine Verbindung der Formel (IV): 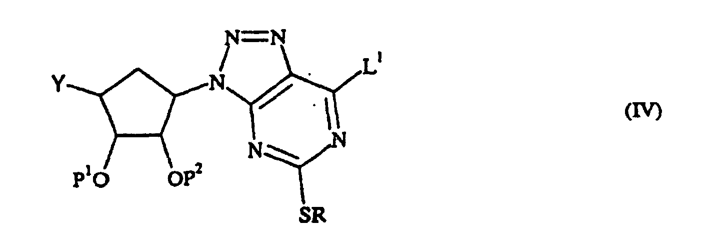
wobei Y für CO₂H, CO₂R' oder CONR⁵R⁶ und R, R⁵, R⁶, P¹ und P² wie oben definiert sind und L¹ für eine Abgangsgruppe steht und R' für eine C₁₋₆-Alkyl- oder Benzylgruppe steht, mit einem Amin NHR¹R² oder einem Salz von NHR¹R², wobei R² wie oben definiert ist, umsetzt
und gegebenenfalls im Anschluß an (a) bzw. (b) und in beliebiger Reihenfolge:
• ein oder mehrere funktionelle Gruppen in andere funktionelle Gruppen umwandelt
• gegebenenfalls vorhandene Schutzgruppen entfernt
• ein pharmazeutisch unbedenkliches Salz oder Solvat bildet.

## Revendications

1. Composé de formule (I) 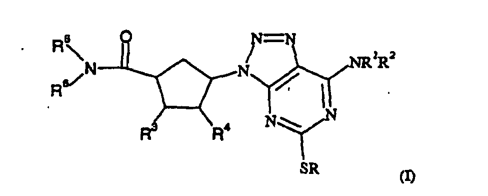 dans laquelle :
R est un alkyle en C₁₋₆ éventuellement substitué par un ou plusieurs atomes d'halogène ;
R¹ est un alkyle en C₁₋₄ ;
R² est un alkyle en C₁₋₆ ou un groupe cycloalkyle en C₃₋₈ éventuellement substitué par un phényle éventuellement substitué par un ou plusieurs substituants choisis parmi un halogène, SO₂NR⁸R⁹ , et un alkyle en C₁₋₆ qui est éventuellement substitué par un ou plusieurs atomes d'halogène ;
R³ et R⁴ sont tous les deux un hydroxy ;
R⁵ est un hydrogène ou un alkyle en C₁₋₆, et R⁶ est un alkyle en C₁₋₆ éventuellement substitué par un cycloalkyle en C₃₋₆ ou R⁶ est un cycloalkyle en C₃₋₆, ou R⁵ et R⁶ forment, conjointement avec l'atome d'azote auquel ils sont liés, un noyau saturé à 5 - 7 chaînons ;
R⁸ et R⁹ sont indépendamment un hydrogène ou un alkyle en C₁₋₆ ;
ou un sel ou solvate pharmaceutiquement acceptable de celui-ci.

2. Composé selon la revendication 1, dans lequel R est un méthyle ou un propyle.

3. Composé selon la revendication 1 ou 2, dans lequel R¹ est un méthyle.

4. Composé selon l'une quelconque des revendications 1 à 3, dans lequel R² est un cyclopropyle éventuellement substitué par un phényle, un 4-chlorophényle, un 3,4-difluorophényle, un 4-(aminosulfonyl)phényle ou un 4-(méthylaminosulfonyl)phényle.

5. Composé selon l'une quelconque des revendications 1 à 4, dans lequel R⁵ est un hydrogène et R⁶ est un alkyle en C₁₋₆ éventuellement substitué par un hydroxy ou un fluor.

6. Composé selon l'une quelconque des revendications 1 à 5, présentant la stéréochimie suivante

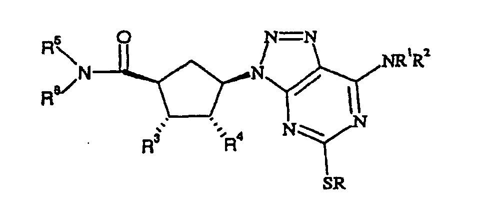

7. Composé selon les revendications 1 à 6, à savoir :
le [1*S*-[1α,2β,3β,4α(1*S**,2*R**)]]-*N*-éthyl-2,3-dihydroxy-4-[7-[méthyl (2-phénylcyclopropyl) amino]-5-(propylthio)-3*H*-[1,2, 3]triazolo[4, 5-*d*]pyrimidin-3-yl]cyclopentane-carboxamide,
le [1*S*-[1α,2β,3β,4α(1*S**,2*R**)]]-*N*-éthyl-2,3-dihydroxy-4-[7-[méthyl (2-phénylcyclopropyl) amino]-5-(méthylthio) -3*H*-[1, 2, 3]triazolo[4, 5-*d*]pyrimidin-3-yl]cyclopentane-carboxamide,
le [1*S*-[1α,2β,3β,4α(1*S**,2*R**)]]-2,3-dihydroxy-*N*-(2-hydroxyéthyl)-4-[7-[méthyl(2-phénylcyclopropyl)amino]-5-(propylthio) -3*H*-[1, 2, 3]triazolo[4, 5-*d*]pyrimidin-3-yl]-cyclopentanecarboxamide,
le [1*S*-[1α,2β,3β,4α(1*S**,2*R**)]]-2,3-dihydroxy-4-[7-[méthyl (2-phénylcyclopropyl) amino]-5-(propylthio)-3*H*-[1,2, 3]triazolo[4, 5-*d*]pyrimidin-3-yl]cyclopentane-carboxamide,
le [1*S*-[1α,2β,3β,4α(1*S**,2*R**)]]-4-[7-[[2-(3,4-difluorophényl)cyclopropyl]méthylamino]-5-(propylthio)-3*H*-[1, 2, 3]triazolo[4, 5-*d*]pyrimidin-3-yl]-*N*-éthyl-2, 3-dihydroxycyclopentanecarboxamide,
le [1*S*-[1α,2β,3β,4α(1*S**,2*R**)]]-4-[7-[[2-(4-chlorophényl)-cyclopropyl]méthylamino]-5-(propylthio)-3*H*-[1,2, 3]triazolo[4,5-*d*]pyrimidin-3-yl]-*N*-éthyl-2, 3-dihydroxycyclopentanecarboxamide,
le [1*S*-[1α,2β,3β,4α(1*S**,2*R**)]]-4-[7-[[2-(4-aminosulfonyl-phényl)cyclopropyl]méthylamino]-5-(propylthio)-3*H*-[1,2, 3]triazolo[4, 5-*d*]pyrimidin-3-yl]-*N*-éthyl-2,3-dihydroxycyclopentanecarboxamide,
le [1*S*-[1α,2β,3β,4α(1*S**,2*R**)]]-*N*-éthyl-2,3-dihydroxy-4-[7-[méthyl[2-(4-méthylaminosulfonylphényl)cyclopropyl]-amino]-5-(propylthio) -3*H*-[1, 2, 3]triazolo[4, 5-*d*]pyrimidin-3-yl]cyclopentanecarboxamide,
le [1*S*-[1α,2β,3β,4α(1*S**,2*R**)]]-*N*-(2-fluoroéthyl)-2,3-dihydroxy-4-[7-[méthyl(2-phénylcyclopropyl)amino]-5-(propylthio)-3*H*-[1,2,3]triazolo[4,5-*d*]pyrimidin-3-yl)-cyclopentanecarboxamide ou leurs sels ou solvates pharmaceutiquement acceptables.

8. Composition pharmaceutique comprenant un composé selon l'une quelconque des revendications 1 à 7, en combinaison avec un diluant, un adjuvant ou un support pharmaceutiquement acceptable.

9. Composé selon l'une quelconque des revendications 1 à 8, destiné à être utilisé en thérapie.

10. Procédé de préparation d'un composé de formule (I) comprenant :
a) la réaction d'un composé de formule (II) : 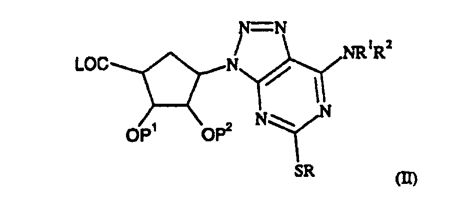 dans laquelle R, R¹ et R² sont tels que définis dans la formule (I) ou sont leurs dérivés protégés, P¹ et P² sont un hydrogène ou des groupes protecteurs et L est un groupe partant, avec un composé de formule (III) :
R⁵R⁶NH (III)
dans laquelle R¹ et R² sont tels que définis dans la formule (I), ou
(b) la réaction d'un composé de formule (IV) : 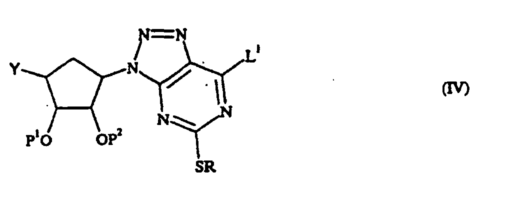 dans laquelle Y est CO₂H, CO₂R' ou CONR⁵R⁶ et R, R⁵, R⁶, P¹ et P² sont tels que définis ci-dessus et L¹ est un groupe partant, et R' est un groupe alkyle en C₁₋₆ ou benzyle, avec une aminé NHR¹R² ou un sel de NHR¹R² dans laquelle R² est tel que défini ci-dessus,
et éventuellement après (a) ou (b) et dans un ordre quelconque :
• la transformation d'un ou de plusieurs groupes fonctionnels en d'autres groupes fonctionnels
• l'élimination de tous les groupes protecteurs
• la formation d'un sel ou solvate pharmaceutiquement acceptable.
